# EUROPEAN PATENT APPLICATION

(11) **EP 0 727 488 A1**
(43) Date of publication of application: **21.08.1996**
(21) Application number: 95921978.3
(22) Date of filing: 16.06.1995
(51) Int. Cl.: C12N 15/12, C07K 14/705, C12N 5/10

(54) **GLUCAN ELICITOR RECEPTOR AND DNA CODING FOR THE SAME**

(30) Priority: 17.06.1994 JP 136100/94
(71) Applicant: KIRIN BEER KABUSHIKI KAISHA, Chuo-Ku, Tokyo 104 (JP)
(72) Inventor: YOSHIKAWA, Masaaki +di, . (JP); KAKITANI, Makoto Kirin Beer K. K. Kiban Gijutsu, Yokohama-shi Kanagawa 236 (JP); UMEMOTO, Naoyuki Kirin Beer K. K. Kiban Gijutsu, Yokohama-shi Kanagawa 236 (JP); ISHIDA, Isao Kirin Beer K. K. Kiban Gijutsu, Yokohama-shiKanagawa 236 (JP); IWAMATSU, Akihiro Kirin Beer K. K. Kiban Gijutsu, Yokohama-shi Kanagawa 236 (JP)
(74) Representative: Kolb, Helga, Dr. Dipl.-Chem.
(86) International application number: JP9501206
(87) International publication number: WO9535371

(57) **Abstract**

A glucan elicitor receptor having substantially the amino acid sequence represented by SEQ ID NO: 1 in the Sequence Listing; a DNA or a fragment thereof containing a base sequence coding for the glucan elicitor receptor having substantially the amino acid sequence represented by SEQ ID NO: 1 in the Sequence Listing; a DNA or a fragment thereof integrated into plasmid pER 23-1 and containing a base sequence coding for the glucan elicitor receptor; a vector containing the DNA or the fragment thereof; and a plant cell containing the DNA or the fragment thereof incorporated thereinto.

## Description

### TECHNICAL FIELD

The present invention relates to glucan elicitor receptor (hereinafter sometimes referred to as "ER"), DNA molecules coding for ER, vectors containing the DNA molecules and plant cells transformed with the DNA molecules. The present invention relates more specifically to ER derived from a soybean root plasma membrane fraction, DNA molecules coding for ER, vectors containing the DNA molecules and plant cells transformed with the DNA molecules.

### BACKGROUND ART

It is known that plants synthesize and accumulate an antibiotic agent called phytoalexin in response to infection with pathogens (M. Yoshikawa (1978) Nature 257: 546). Some plant pathogens were found to have the substances that induce them to perform such a resistance reaction (N.T. Keen (1975) Science 187: 74), which are called "elicitors". The biochemical process from the infection of plants with pathogens to the synthesis and accumulation of phytoalexin is believed to be as follows:

When the mycelium of a pathogen invades a plant cell, glucanase in the plant cell works so as to cleave polysaccharides on the surface of the pathogen mycelial wall, thereby liberating an elicitor. If the elicitor binds to a receptor in the plant cell, a second messenger which plays a role in signal transduction is produced. The signal transduction substance is incorporated in the nucleus of the plant cell and activates the transcription of the genes coding for phytoalexin synthesize enzymes to induce a phytoalexin synthesis. At the same time, the phytoalexin degradation is inhibited. As a result, phytoalexin is efficiently accumulated.

Phytoalexin playing an important role in the resistance of soybean is called glyceollin and its structure has been determined (M. Yoshikawa et al. (1978) Physiol. Plant. Pathol. 12: 73). Elicitor is a polysaccharide of glucose and reported to be a glucan having β-1,6 and β-1,3 linkages (J.K. Sharp et al. (1984) J. Biol. Chem. 259: 11321, M. Yoshikawa (1990) Plant Cell Technology 1.2: 695). A specific receptor for glucan elicitor derived from a soybean pathogenic mold fungus Phytophthora megasperma f. sp. glycinea is believed to be a protein which plays an important role in the synthesis and accumulation of the antibiotic agent glyceollin. The method for the purification of the ER specific to this elicitor has been disclosed (E.G. Cosio et al., (1990) FEBS 264: 235, E.G. Cosio et al. (1992) Eur. J. Biochem. 204: 1115, T.Frey et al. (1993) Phytochemistry 32: 543). However, the amino acid sequence of the ER has not been determined and the gene coding therefor is not yet known.

An object of the present invention is to provide a glucan elictor receptor.

Another object of the present invention is to provide DNA molecules coding for the glucan elicitor receptor.

A further object of the present invention is to provide vectors containing DNA molecules coding for the glucan elicitor receptor.

A still further object of the present invention is to provide plant cells transformed with DNA molecules coding for the glucan elicitor receptor.

### DISCLOSURE OF THE INVENTION

As a result of the various studies conducted to solve the above problems, the inventors succeeded in purifying a soybean root-derived ER and cloning the ER gene from a soybean cDNA library, thereby accomplishing the present invention. The present invention provides a glucan elicitor receptor having an amino acid sequence as substantially shown in SEQ ID NO:1. The present invention also provides DNA molecules containing nucleotide sequences coding for a glucan elicitor receptor having an amino acid sequence as substantially shown in SEQ ID NO:1, and fragments thereof. The present invention further provides DNA molecules containing nucleotide sequences coding for the glucan elicitor receptor which are incorporated in plasmid pER23-1, and fragments thereof. The present invention still further provides vectors containing DNA molecules coding for the glucan elicitor receptor and plant cells transformed with DNA molecules coding for the glucan elicitor receptor.

### BRIEF DESCRIPTION OF THE DRAWINGS

Figure 1 shows SDS-polyacrylamide gel electrophoresis patterns of three purification steps.

Figure 2 shows the maps of plasmids pER23-1 and pER23-2.

Figure 3 shows the procedure for constructing plasmid pKV1-ER23.

Figure 4 shows a transient increase in intracellular Ca²⁺ concentration after the addition of an elicitor to cultured soybean cells.

Figure 5 shows a transient increase in intracellular Ca²⁺ concentration after the addition of an elicitor to transformed cultured tobacco cells.

Figure 6 shows elicitor-binding activities of full- or partial length ER expressed in E. coli.

Figure 7 shows the inhibition of the binding of an elicitor with an elicitor binding protein in a soybean cotyledon membrane fraction by an antibody against an elicitor-binding domain.

Figure 8 shows the inhibition of an elicitor-induced phytoalexin accumulation in soybean cotyledons by an antibody against an elicitor-binding domain.

### PREFERRED EMBODIMENTS OF CARRYING OUT THE INVENTION

The glucan elicitor receptor of the present invention is a protein having a function as a receptor for glucan elicitors derived from plant phatogens, particularly Phytophthora. More specifically, it is a protein which binds to the glucan elicitor produced by the cleavage of parts of the pathogen mycelial wall with β-1,3-glucanase in plant cells at the time of invasion of plant pathogens, particularly microorganisms belonging to genus Phytophthora and which subsequently orders the microsomes and nucleus to produce an increased amount of phytoalexin in the plant cells. The glucan elicitor receptor of the present invention have an amino acid sequence as substantially shown in SEQ ID NO:1. The "amino acid sequence as substantially shown in SEQ ID NO:1" includes amino acid sequences as shown in SEQ ID NO: 1 in which there may be the deletion, replacement or addition of an amino acid(s), provided that they maintain the function of a glucan elicitor receptor.

The glucan elicitor receptor of the present invention can be produced, for example, by a partially modified Cosio's method (E.J.B. (1992) 204: 1115). Briefly, the roots, leaves and stems of soybean, preferably variety green homer are homogenized and a membrane fraction is collected from the resulting slurry, purified by ion-exchange chromatography and further purified by affinity chromatography using an elicitor as a ligand. The elicitor used in the affinity chromatography is preferably derived from Phytophthora megasperma f. sp. glycinea race 1 (ATCC34566) because it shows incompatibility for green homer (i.e., resistance to the pathogen).

The amino acid sequence of the glucan elicitor receptor thus prepared can be determined as follows:

The purified ER is transferred on a PVDF membrane (Millipore Co.) by electroblotting and digested with lysylendopeptidase (AP-I). The fragmented peptides are recovered from the PVDF membrane and fractionated by reversed-phase HPLC (µ-Bondasphere 5 µ C8). The peak fractions are analyzed with a gas-phase protein sequencer (Applied Biosystems Co.).

The ER of the present invention is useful in the elucidation of resistance mechanism of plants to fungi and the development of elicitor derivatives capable of inducing resistance to fungi, and it can be used as an antigen for the production of antibodies against ERs.

The present invention encompasses DNA molecules containing nucleotide sequences coding for a glucan elicitor receptor, and fragments thereof. The DNA molecules of the present invention have preferably at least one stop codon (e.g., TAG) adjacent to the 3' end.

More specifically, the present invention encompasses DNA molecules containing nucleotide sequences coding for a glucan elicitor receptor having an amino acid sequence as substantially shown in SEQ ID NO: 1, and fragments thereof. The "DNA molecules containing nucleotide sequences coding for a glucan elicitor receptor" include all degenerate isomers. The term "degenerate isomers" means DNA molecules coding for the same polypeptide with different degenerate codons. If a DNA molecule having the nucleotide sequence of SEQ ID NO:2 is taken as an example, a DNA molecule in which a codon for any amino acid, e.g., AAC for Asn is changed to a degenerate codon AAT is called a degenerate isomer. Examples of such degenerate isomers include DNA molecules containing the nucleotide sequence shown in SEQ ID NO: 2.

In another aspect, the present invention provides DNA molecules containing nucleotide sequences coding for a glucan elicitor receptor, which are incorporated in plasmid pER23-1, and fragments thereof. E. coli DH5α EKB633 transformed with plasmid pER23-1 was deposited with the National Institute of Bioscience and Human-Technology, the Agency of Industrial Science and Technology, on June 15, 1994 under Accession Number FERM BP-4699.

The fragments of the DNA molecules containing nucleotide sequences coding for the glucan elicitor receptor of the present invention may contain a nucleotide sequence encoding the amino acid sequence of SEQ ID NO:1.

The DNA molecules of the present invention which contain nucleotide sequences coding for a glucan elicitor receptor and fragments thereof may optionally bind to an ATG codon for initiation methionine together with a translation frame in the upstream portion toward the 5' end and also bind to other DNA molecules having appropriate lengths as non-translation regions in the upstream portion toward the 5' end and the downstream portion toward the 3' end.

The DNA molecules of the present invention which contain nucleotide sequences coding for a glucan elicitor receptor and fragments thereof can be present typically in the form of parts of constituents of plasmid or phage DNA molecules or in the form of parts of constituents of plasmid, phage or genomic DNA molecules which are introduced into microorganisms (particularly, bacteria including E. coli and Agrobacterium), phage particles or plants.

In order to express stably the DNA molecules coding for a glucan elicitor receptor and fragments thereof in plants, a promoter, a DNA molecule (ATG) encoding the initiation codon and a terminator may be added to the DNA molecules of the present invention and fragments thereof in appropriate combinations. Examples of the promoter include the promoter of genes encoding ribulose-1,5-biphosphate carboxylase small subunit (Fluhr et al., Proc. Natl. Acad. Sci. USA (1986) 83: 2358), the promoter of a nopaline synthase gene (Langridge et al., Plant Cell Rep. (1985) 4:355), the promoter for the production of cauliflower mosaic virus 19S-RNA (Guilley et al., Cell (1982) 30:763), the promoter for the production of cauliflower mosaic virus 35S-RNA (Odell et al., Nature (1985) 313:810) and the like. Examples of the terminator include the terminator of a nopaline synthase gene (Depicker et al., J. Mol. Appl. Gen. (1982) 1:561) and the terminator of an octopine synthase gene (Gielen et al., EMBO J. (1984) 3:835).

The DNA molecule containing a nucleotide sequence coding for a glucan elicitor receptor can be obtained by a method comprising the steps of chemically synthesizing at least a part of the DNA molecule according to a conventional procedure of synthesis of nucleic acids and obtaining a desired DNA molecule from an appropriate cDNA library using the synthesized DNA molecule as a probe by a conventional method, for example, an immunological method or a hybridization method. Some plasmids, various kinds of restriction enzymes, T4DNA ligase and other enzymes for use in the above method are commercially available. The DNA cloning, the construction of plasmids, the transfection of a host, the cultivation of the transfectant, the recovery of DNA molecules from the culture and other steps can be performed by the methods described in Molecular Cloning, J. Sambrook et al., CSH Laboratory (1989), Current Protocols in Molecular Biology, F. M. Ausubel et al., John Wiley & Sons (1987) and others.

More specifically, the DNA molecules of the present invention which contain nucleotide sequences coding for a glucan elicitor receptor can be obtained as follows:

Two kinds of partial amino acid sequences are selected from the amino acid sequences of a glucan elicitor receptor. Primers consisting of combinations of all bases which can encode the C terminus of the selected partial sequence and primers consisting of combinations of `all bases which can encode the N terminus of the selected partial sequence are prepared. These synthesized primers are used as mixed primers to perform two PCR using DNA molecules of an appropriate soybean cDNA library as a template. Subsequently, two amplified fragments of given lengths whose amplification is expected (these fragments correspond to DNA molecules encoding the above two partial amino acid sequences) are picked up and the nucleotide sequences thereof are determined. On the basis of the determined nucleotide sequences, a primer having nucleotide sequences coding for the C terminus of an amino acid partial sequence positioned at the C terminal side of the glucan elicitor receptor and a primer having nucleotide sequences coding for the N terminus of an amino acid partial sequence positioned at the N terminal side of the glucan elicitor receptor are synthesized. These two synthesized primers are used to perform a PCR using the DNA molecules of the above soybean cDNA library as a template. The resulting amplified fragments are used as probes to hybridize the aforementioned soybean cDNA library, thereby yielding DNA molecules containing nucleotide sequences coding for the glucan elicitor receptor.

The obtained DNA molecules containing nucleotide sequences coding for the glucan elicitor receptor can be sequenced by any known methods, for example, the Maxam-Gilbert method (Methods Enzymol., 65:499, 1980), a dideoxynucleotide chain termination method using M13 phage (J. Messing, et al., Gene, 19:269, 1982) and the like.

Since the results of various studies on glucan elicitor suggest that a glucan elicitor receptor plays an important role in resistance to fungi in plants, it is expected that the DNA molecules coding for glucan elicitor receptor of the present invention and fragments thereof can impart fungal resistance to plants if they are introduced and expressed in plant cells (particularly higher plant cells) which have no glucan elicitor receptor according to a conventional procedure. It has been proposed that fungi capable of infecting plants have generally suppressors, thereby acquiring an ability to suppress the fungal resistance of the plants. It is expected that new plants having resistance to fungi can be developed by introducing and expressing the DNA molecules coding for glucan elicitor receptor of the present invention and fragments thereof such that the ER works or by modifying the DNA molecules and fragments thereof or by regulating their expression amounts. Moreover, if the DNA molecules of the present invention and fragments thereof are introduced and expressed in plant cells, particularly in higher plant cells, together with fungal resistance enhancing genes or characters such as the gene of glucanase which imparts fungal resistance to plants, it is expected that higher fungal resistance can be imparted to plants than in the case where the gene of glucanase is introduced.

Vectors used for introducing the DNA molecules coding for the glucan elicitor receptor and fragments thereof may be constructed such that the glucan elicitor receptor can be stably expressed in plants. More specifically, a promoter, a DNA molecule encoding the initiation codon (ATG) and a terminator may be added to the DNA molecules coding for the glucan elicitor receptor and fragments thereof in appropriate combinations. Examples of the promoter include the promoter of genes encoding ribulose-1,5-biphosphate carboxylase small subunit (Fluhr et al., Proc. Natl. Acad. Sci. USA (1986) 83:2358), the promoter of a nopaline synthase gene (Langridge et al., Plant Cell Rep. (1985) 4: 355), the promoter for the production of cauliflower mosaic virus 19S-RNA (Guilley et al., Cell (1982) 30:763), the promoter for the production of cauliflower mosaic virus 35S-RNA (Odell et al., Nature (1985) 313:810) and the like. Examples of the terminator include the terminator of a nopaline synthase gene (Depicker et al., J. Mol. Appl. Gen. (1982) 1:561) and the terminator of an octopine synthase gene (Gielen et al., EMBO J. (1984) 3:835).

The DNA molecules coding for a glucan elicitor receptor and fragments thereof can be introduced into plant cells by any usual known methods, for example, the method described in "Plant genetic transformation and gene expression; a laboratory manual", J. Draper, et al. eds., Blackwell Scientific Publications, 1988. Examples of the methods include biological methods such as those using viruses or Agrobacteria and physicochemical methods such as electroporation, a polyethylene glycol method, microinjection and the like.

The present invention will now be explained in greater detail with reference to the following examples which are by no means intended to limit the scope of the present invention.

### Example 1

### Purification of soybean root-derived ER

### 1) Measurement of glucan elicitor binding activity of ER

A complex of an elicitor (average molecular weight: 10,000) and tyramine (TOKYO KASEI KOGYO CO., LTD.) was synthesized by the method of Jong-Joo Cheong (The Plant Cell (1991) 3: 127). The elicitor-tyramine complex was labelled with iodine using chloramine T.

A sample (protein amount < 500 µg) was suspended in 500 µl of an assay buffer (50 mM Tris-HCl pH7.4, O.1 M saccharose, 5 mM MgCl₂, 1mM PMSF and 5 mM EDTA) and incubated at 0°C for 2 hours. The iodine-labelled elicitor-tyramine complex in an amount of 7.0 nM (70 Ci/mmol, the number of moles is calculated on the assumption that the molecular weight of the elicitor is 10,000, and this applies to the following description) was added to the suspension and the mixture was incubated at 4 °C for 2 hours. The reaction solution was filtered through Whatman GF/B as treated with a 0.3% aqueous solution of polyethylene imine for at least 1 hour. The residue was washed 3 times with 5 ml of an ice-cold buffer (10 mM Tris-HCl pH 7.0, 1 M NaCl, 10 mM MgCl₂). The radio activity retained on the filter was counted with a gamma counter (count A). In order to eliminate the effect of non-specific binding, the same procedure as above was performed except that 17 µM of the elicitor was added to the same sample, the mixture was suspended in the assay buffer and the suspension was incubated at 0 °C for 2 hours. The obtained count was subtracted from the count A to give a count (Δ cpm) of elicitor-specific binding. The resulting count (Δ cpm) was divided by the total number of counts and then multiplied by the total amount of elicitor used in the experiment to calculate the amount of the elicitor-binding protein (in moles).

The purity of ER was checked by the above method.

### 2) Purification of soybean root-derived ER

Soybean (Glycine max cv. Green Homer) seeds (Takayama Seed Co.) were cultured on vermiculite for 1 week and then aquicultured for 15 days to harvest roots (about 40 kg, wet weight). The harvested roots were stored at -80 °C until they were used for the purification of ER. An ice-cold buffer (25 mM Tris-HCl pH 7.0, 30 mM MgCl₂, 2 mM dithiothreitol, 2.5 mM potassium metabisulfite and 1 mM PMSF) was added to the roots (2 kg, wet weight) in an amount of 1.25 L and the mixture was homogenized with a Waring Blender for 2 minutes.

The resulting slurry was filtered through a Miracloth (Calbiochem Co.) and the filtrate was centrifuged at 9,000 rpm at 4 °C for 15 minutes. The supernatant was ultracentrifuged at 37,000 rpm at 4°C for 20 minutes. The precipitate was suspended in 160 ml of an ice-cold buffer (25 mM Tris-HCl pH 7.4, 0.1 M sucrose, 5 mM MgCl₂, 1 mM PMSF and 5 mM EDTA) to give a membrane fraction. An ampholytic detergent ZW3-12 (Boehringer Co.) was added to the membrane fraction to give a final concentration of 0.25% for solubilization of ER from the membrane fraction and the mixture was stirred at 8 °C for 30 minutes. The resulting mixture was ultracentrifuged at 37,000 rpm at 4 °C for 20 minutes to collect the supernatant containing the solubilized ER (soluble fraction). The soluble fraction (165 ml) was dialyzed against 2 l of a buffer (50 mM Tris-HCl pH 8.0, 0.2% ZW3-12, 4 °C) 4 times. Five milliliters of Protrap (TAKARA SHUZO CO., LTD.) was added to the sample and the mixture was stirred at 8 °C for 30 minutes to remove proteases from the sample and to stabilize ER. The resulting mixture was centrifuged at 2,800 rpm at 4 °C for 2 minutes to collect the supernatant. The obtained supernatant (160 ml) was concentrated to about 50 ml using an ultrafiltration membrane YM-10 (Amicon Co.) and the concentrate was dialyzed against an A buffer (50 mM Tris-HCl pH 8.0, 0.1 M sucrose, 5 mM MgCl₂, 1 mM PMSF, 5 mM EDTA and O.2% ZW3-12, 4 °C).

The dialysate was applied to Q-Sepharose HP 26/10 (Pharmacia Co.) and ER was eluted in a linear gradient of 0-1 M NaCl (Q-Sepharose active fraction). The ER was eluted at a NaCl concentration of about 0.45 M. The Q-Sepharose active fraction was diluted 3 folds with A buffer and the diluted fraction was applied to Mono Q 10/10 (Pharmacia Co.). The ER was eluted in a linear gradient of 0-1 M NaCl (Mono Q active fraction, 8 ml). The ER was eluted at a NaCl concentration of about 0.25 M.

The ER was purified with an affinity gel using an elicitor as a ligand as follows:

Elicitor was prepared according to N.T. Keen with some modifications (Plant Physiol. (1983) 71: 460, Plant Physiol. (1983) 71: 466). Briefly, the mycelial wall of pathogenic Phytophthora megasperma f. sp. glycinea race 1 (ATCC34566) was treated with zymolyase 100T (KIRIN BREWERY CO., LTD.) to liberate an elicitor. After the treatment, Zymolyase 100T was eliminated by the adsorption on CM-cellulose packed in a column. The resulting passage-through fraction was purified with a gel permeation chromatography G-75 (Pharmacia Co.) to collect an elicitor fraction whose average molecular weight was 10,000 Da. The glyceollin-inducing elicitor activity of the collected fraction was determined by the method of M. Yoshikawa (Nature (1978) 257:546). The addition of 8 µg of the elicitor to soybean cotyledons resulted in the induction of about 550 µg of glyceollin after 24 hours incubation.

In order to eliminate non-specific adsorption on the gel carrier, Mono Q active fraction was collected and stirred with about 33 mg of maltose-coupled glass gel (bed volume: about 100 µl) at 8 °C for 1 hour. The gel was precipitated by centrifugation (1,000 rpm, 4 °C, 2 minutes) to collect the supernatant (maltose-coupled glass gel passage-through fraction). The maltose-coupled glass gel was prepared by the method of A.M. Jeffrey et al. (Biochem. Biophys. Res. Commun. (1975) 62: 608). Briefly, 120 mg of maltose and 6 g of Glass Aminopropyl (Sigma Co.) were suspended in 36 ml of H₂O and the suspension was stirred at room temperature overnight. To the resulting suspension was added 36 ml of ethanol. Immediately thereafter a solution of sodium borohydride (864 mg) in ethanol (72 ml) was added to the mixture. The resulting mixture was sonicated for 2 minutes and stirred at room temperature for 5 hours. Water (288 ml) was added to the reaction mixture and the resulting mixture was cooled with ice and adjusted to pH 5.6 with acetic acid. The gel was washed with about 1.8 L of H₂O to remove the free maltose. Maltose contained in the washing solution was determined quantitatively by the method of J. H. Roe (J. Biol. Chem. (1955) 212:335) using an anthrone reagent. An amount of the gel-coupled maltose was estimated from the amount of the maltose contained in the washing solution. As a result, it was found that 60 mg of maltose was coupled to 6 g of Glass Aminopropyl.

About 17 mg of the elicitor-coupled glass gel (bed volume: about 50 µl) was added to 8 ml of the maltose-coupled glass gel passage-through fraction and the mixture was stirred gently at 8 °C overnight. The gel was collected by centrifugation (1,000 rpm, 4 °C, 2 minutes) and washed with 2 bed volumes of A buffer 2 times. The gel was washed additionally with 4 bed volumes of 0.1% SDS 3 times to collect gel-coupled ER (elicitor-coupled glass gel eluted fraction). The elicitor-coupled glass gel was prepared by the method of A.M. Jeffrey et al. (Biochem. Biophys. Res. Commun. (1975) 62: 608). Briefly, elicitor (37 mg) and Glass Aminopropyl (490 mg) were suspended in 6 ml of H₂O and stirred at room temperature overnight. Ethanol (6 ml) was added to the suspension and a solution of sodium borohydride (144 mg) in ethanol (12 ml) was added immediately thereafter. The mixture was sonicated for 2 minutes and stirred at room temperature for 5 hours. To the resulting mixture was added 48 ml of H₂O. The mixture was cooled with ice and adjusted to pH 5.6 with acetic acid. The free elicitor was determined quantitatively with an anthrone reagent. The amount of the gel-coupled elicitor was estimated from the amount of the free elicitor contained in the washing solution. As a result, it was found that 34 mg of the elicitor was coupled to 490 mg of Glass Aminopropyl.

The protein and ER amounts in the above steps for purification are summarized in Table 1.

**Table 1**

| Protein and ER Amounts in the Steps for Purification (Soybean roots weighing 40 kg on a wet basis were used as a starting material) | | |
|---|---|---|
| | Protein (mg) | ER (pmol) |
| Membrane Fraction | 17900 | 30 |
| Soluble Fraction | 2000 | 214 |
| Q-Sepharose Active Fraction | 190 | 205 |
| Mono Q Active Fraction | 49 | 233 |
| Maltose-Coupled Glass Gel Passage-through Fraction | 45 | 220 |
| Elicitor-Coupled Glass Gel Eluted Fraction | 0.004* | 45 |

| | | |
|---|---|---|
| * : Estimated from the band intensity obtained by silver stain after SDS-PAGE. | | |

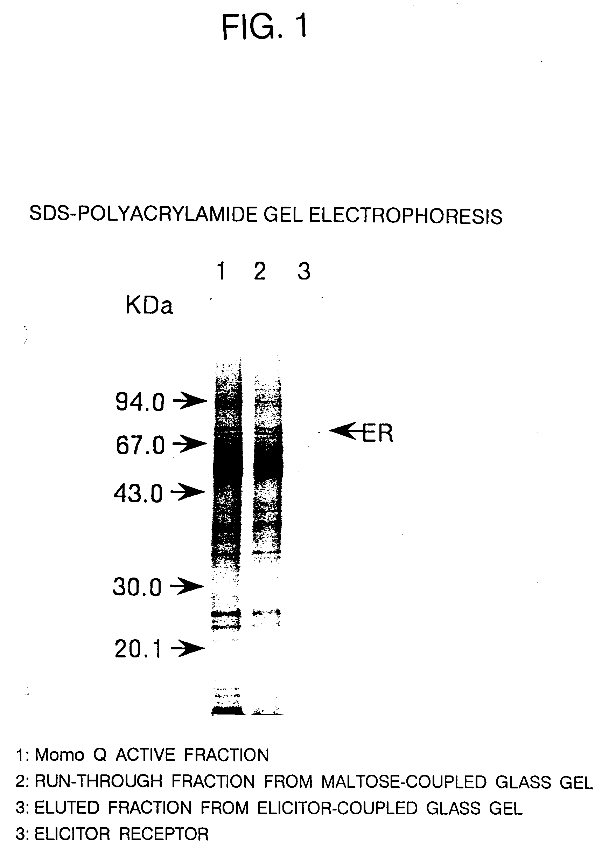

The Mono Q active fraction, passage-through fraction from maltose-coupled glass gel and eluted fraction from elicitor-coupled glass gel (10 µl each) were electrophoresed on an electrophoretic gradient gel, SDS-PAGE plate 10/20 (Daiich Kagaku Yakuhin Co.) and stained with silver (Daiich Kagaku Yakuhin Co.) The electrophoresis patterns are shown in Figure 1. In Figure 1, lane 1 is the Mono Q active fraction, lane 2; the passage-through fraction from the maltose-coupled glass gel and lane 3; the eluted fraction from the elicitor-coupled glass gel. Figure 1 shows that the ER bands were detected at a molecular weight of about 70,000 Da.

The protein of about 70,000 in molecular weight was labelled with iodine-125 by using an ¹²⁵I-labelled complex of a photoaffinity reagent SASD (Pierce Co.) and the elicitor. The SDS-PAGE band of the membrane fraction was transferred on a PVDF membrane by western blotting and incubated with the same ¹²⁵I-labelled elicitor as used in measuring the elicitor-binding activity of ER on the PVDF membrane so that the protein of about 70,000 Da in molecular weight was labeled with iodine-125. These facts reveal that the protein of about 70,000 Da in molecular weight had an elicitor-binding activity.

About 4 µg of ER was purified from about 40 kg by wet weight of the soybean root by the above method.

### 3) Analysis of ER-fragmented peptides

The ER was fragmented by protease digestion to peptides. The amino acid sequences of the fragmented peptides were determined by the method of Iwamatsu (Akihiro Iwamatsu, Seikagaku (1991) 63: 139, A. Iwamatsu, Electrophoresis (1992) 13: 142). A solution of the ER purified by the above method was concentrated to about 100 µl with Centricon-30 (Amicon Co.) and subjected to a 10-20% polyacrylamide SDS electrophoresis. The resulting protein bands were transferred on a PVDF membrane (Millipore Co.) with an electroblotting apparatus (Sartrius Co.). The bands transferred on the PVDF membrane were stained with 0.1% Ponceau S (Sigma Co.)/1% acetic acid. The main band of 70,000 Da in molecular weight was sectioned and decolored with 0.5 mM NaOH. This band was reductively S-carboxymethlated. Lysylendopeptidase (AP-1) was added to the resulting band at an enzyme:substrate (mol:mol) ratio of 1:100 and the mixture was reacted at 30 °C for 16 hours. The resulting fragmented peptides were applied to a µ-Bondasphere 5 µ C8-300 Å (2.1 x 150 mm, Waters) column equilibrated with 98% solvent A and 2% solvent B and eluted in a 2-50% linear gradient of solvent B for 30 minutes at a flow rate of 0.25 ml/minute (solvent A: 0.05% TFA solution, solvent B: 0.02% TFA in 2-propanol:acetonitrile=7:3 (v/v)). Eluted peptides were detected by absorbance at 214 nm and each peak fraction was collected manually. The obtained peak fractions were analyzed with a gas-phase protein sequencer (Model 470A of Applied Biosystems). As a result of analysis of all the peak fractions obtained, the following amino acid sequences of the fragmented peptides were clearly determined.
- #1:: Val Asn Ile Gln Thr Asn Thr Ser Asn Ile Ser Pro Gln (N-terminus)
- #5:: Lys Ser Ile Asp Gly Asp Leu Val Gly Val Val Gly Asp Ser
- #6:: Lys Tyr Lys Pro Gln Ala Tyr Ser Ile Val Gln Asp Phe Leu Asn Leu Asp
- #7:: Lys Thr Asp Pro Leu Phe Val Thr Trp His Ser Ile Lys (mix sequence)

### Example 2

### Cloning of soybean ER gene

### 1) Preparation of soybean mRNA

Soybean (Glycine max cv. Green Homer) seeds (Takayama Seed Co.) were cultured on vermiculite for 1 week and aquicultured for 15 days to harvest roots (about 40 kg, wet weight). A portion of the harvested roots was stored at -80°C until it was used. Total RNA was obtained by the method of Ishida (Cell Technology Laboratory Manipulation Manual, Kodansha Scientific). Briefly, the stored roots (28.5 g, wet weight) were ground on a mortar while adding liquid nitrogen. To the obtained powder, 35.6 ml of a GTC solution held at 65°C was added and the mixture was homogenized with a Waring Blender. The resulting suspension was centrifuged at 6,000 rpm at room temperature for 15 minutes to collect 40 ml of the supernatant. The supernatant was layered gently on a cushion solution of cesium in a centrifuge tube and centrifuged at 35,000 rpm at 25 °C for 20 hours. The resulting precipitate was dissolved in 9 ml of TE/0.2% SDS. After phenol/chloroform extraction was conducted 2 times, total RNA (4.37 mg) was recovered by ethanol precipitation.

The obtained total RNA (2.2 mg) was purified with oligotex dT30 (Japan Roche Co.) according to the manual to yield 60 µg of poly(A) +RNA.

### 2) Preparation of soybean cDNA library

cDNA molecules were synthesized from 5 µg of the poly(A)+RNA with a cDNA synthesis kit (Pharmacia Co.) according to the manual. The synthesized cDNA fragments were ligated to lambda phage vector λ gt10 (Stratagene Co.) with T4 ligase (TAKARA SHUZO CO., LTD.). Gigapack (Stratagene Co.) was used to package a DNA mixture into the phage particles to prepare a soybean cDNA library of about 1.5 x 10⁶ pfu. The library was amplified to 160 ml of a soybean cDNA library of 1.6 x 10¹¹ pfu/ml.

Total DNA in the cDNA library was prepared as follows:

Chloroform/isoamylalcohol (24:1) was added to 500 µl of a phage solution (1.6 x 10¹¹ pfu/ml) in an equal amount. The mixture was shaken for 30 seconds and centrifuged to collect the aqueous layer. The aqueous layer was extracted again with chloroform/isoamylalcohol (24:1). To the resulting aqueous layer were added 5 µl of a 3 M sodium acetate solution (pH 5.4) and 125 µl of ethanol and the mixture was centrifuged to collect the precipitate. The precipitate was washed with a 70% ethanol solution and dissolved in a 10 mM Tris-HCl solution (pH 8) containing 1 µg/ml RNase A (Sigma Co.). This solution was used as a PCR template.

### 3) Amplification and Cloning of Soybean ER cDNA Fragments by PCR

The following four oligodeoxynucleotides (mixed primers U5, U7, U10 and U12) were synthesized with an automatic nucleic acid synthesizer (Model 394 of Applied Biosystems Co.) on the basis of the amino acid sequences of the fragmented peptides obtained in Example 1 (#5 and #6):
- Primer U5: 5'-AARAGYATHGAYGGNGA-3'
- Primer U7: 5'-WRTCNCCNACNAC-3'
- Primer U10: 5'-GTNAAYAARATNCARAC-3'
- Primer U12: 5'-ARRTTNAGRAARTCYTC-3'
(R:A/G, Y:C/T, W:A/T, H:A/C/T, N:A/G/T/C)

The total DNA in 0.5 µg of the cDNA library was dissolved in 79 µl of distilled water. Either a combination of primers U5 and U7 or a combination of primers U10 and U12 (100 pmol each) and 0.5 µl of Taq DNA polymerase (TAKARA SHUZO CO., LTD.) were added to 8 µl of 2.5 mM dNTP in 10 µl of a 10 x PCR buffer (attached to Taq DNA polymerase of TAKARA SHUZO CO., LTD.) to give a final amount of 100 µl. PCR reaction was performed with a Gene Amp PCR System 9600 (Perkin-Elmer Co.) by 50 cycles of 1) denaturation at 94 °C x 30 seconds, 2) renaturation at 47°C x 30 seconds and 3) elongation at 72 °C x 1 minute. After the reaction, 15 µl of the reaction solution was electrophoresed on a 15% polyacrylamide gel. The gel was stained with a 0.5 µg/ml ethidium bromide solution for 10 minutes. The bands showing specifically amplified fragments of 40 bp and 47 bp whose amplification was expected were sectioned while observing under UV light. The gel sections were ground with a plastic bar and eluted with an elution buffer (0.5 M ammonium acetate, 10 mM magnesium acetate, 1 mM EDTA and 0.1% SDS) overnight to collect a DNA-containing solution.

The collected DNA fragments were cloned into plasmid pT7Blue(R) with a pT7Blue T-Vector Kit (Novagene Co.). The obtained plasmids p#5-1, 2, and p#6-1, 2, 3, 4, 5, 6, 7, 8 and 9 were sequenced with a fluorescence automatic DNA sequencer (Model 373A of Applied Biosystems Co.). The results showed that the resulting amplified DNA fragments other than the primers also encoded the amino acid sequences of fragmented peptides #5 and #6.

The following two oligodeoxynucleotides (mixed primers U18 and U19) were synthesized with an automatic nucleic acid synthesizer on the basis of the results of the DNA sequencing.
- Primer U18: 5'-AAGTAYAAGCCRCAAGCCTATTCA-3'
- Primer U19: 5'-ATCGCCRACAACMCCAA-3'
(Y and R are as defined above, M:A/C)

The total DNA in 0.5 µg of the cDNA library was dissolved in 79 µl of distilled water. A combination of primers U18 and U19 (100 pmol each) and 0.5 µl of Taq DNA polymerase were added to 8 µl 2.5 mM dNTP in 10 µl of a 10 x PCR buffer to give a final amount of 100 µl. PCR reaction was performed by 40 cycles of 1) denaturation at 94°C x 30 seconds, 2) renaturation at 52°C x 30 seconds and 3) elongation at 72 °C x 1 minute. Fifteen microliters of the reaction solution was electrophoresed on a 1% agarose gel.

The gel was stained with a 0.5 µg/ml ethidium bromide solution for 15 minutes. The band showing a specifically amplified fragment of about 540 bp was sectioned while observing under UV light. The gel section was treated with Gene Clean II (Bio101 Co.) to collect a DNA-containing solution.

The collected DNA fragment was cloned into plasmid pT7Blue(R) with a pT7Blue T-Vector Kit. The obtained plasmid p#5-#6 was sequenced with a fluorescence sequencer. The results showed that the amplified DNA fragment consisted of 539 bp and encoded not only the amino acid sequences of fragmented peptides #5 and #6 at the both sides, but also peptide #7 in the amplified portion.

### 4) Screening and Cloning of Library by Hybridization

Plasmid #5-#6 into which the ER cDNA fragment was cloned was digested with restriction enzymes BamHI and PstI. A DNA fragment of about 540 bp was recovered and used as a probe. The recovered DNA fragment was labelled with [α-³²P] dCTP using a Megaprime DNA labelling system (Amersham Co.) according to the manual and the reaction solution was used in a hybridization experiment.

A phage of the cDNA library was infected with E. coli C600 hfl (Invitrogen Co.) and inoculated in a 10 mg/ml MgCl₂-containing L medium on plates of about 15 cm in diameter to form a total of about 1 x 10⁶ of plaques. The plaques were blotted on a nylon membrane (Hybond-N; Amersham Co.). The membrane was reacted with the ³²P-dCTP labeled ER cDNA fragment and positive phages detected by autoradiography were screened again in the same way to give about 30 phage clones having different signal intensities. Clone λ ER23 having the longest inserted DNA fragment was selected.

The λ phage DNA molecule was purified with a LambdaSorb (Promega Co.) from a solution of the positive clone λ ER23 isolated in the hybridization experiment. Ten microliters of a 10 x EcoRI cleavage buffer (restriction enzyme EcoRI 10 U) was added to 5 µg of the DNA solution to give a total amount of 100 µl and the mixture was reacted at 37 °C overnight. The reaction solution was electrophoresed on a 1% agarose gel. A band of about 2.3 kb was sectioned and treated with a Gene Clean II (Bio101 Co.) to collect a DNA-containing solution. Vector pBluescriptII KS- (0.02 µg) (Stratagene Co.) was cleaved with restriction enzyme EcoRI.

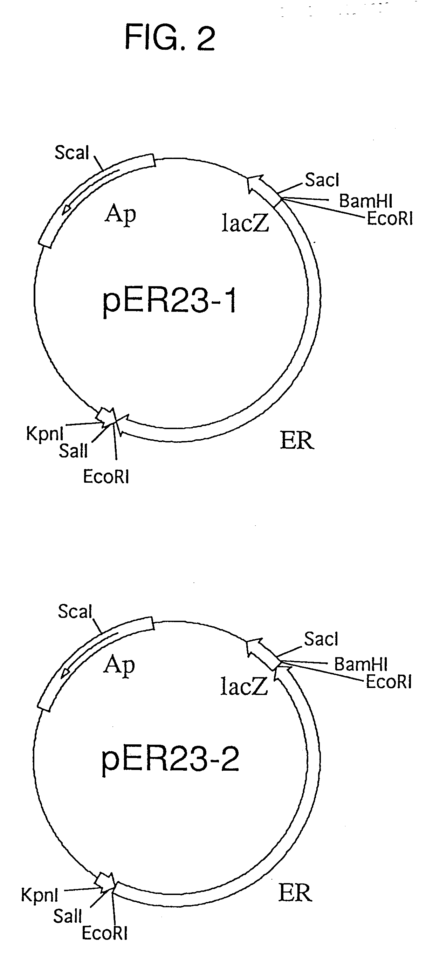

After the two DNA solutions were mixed, 2 µl of a 10 x ligase buffer and 0.2 µl of T4 DNA ligase (TAKARA SHUZO CO., LTD) were added to give a total amount of 20 µl. The mixture was reacted at 16 °C for 4 hours and the reaction mixture solution was used to transform E. coli DH5α (Gibco BRL Co.). A 2% agar plate medium was prepared with 25 ml of an L medium containing 50 µg/ ml ampicillin, 40 µg/ ml IPTG and 40 µg/ ml X-gal. The transformed E. coli was inoculated on the agar plate medium and grown at 37°C overnight. White colonies were selected from the formed colonies and cultured in 3 ml of an L medium containing 50 µg/ ml ampicillin at 37 °C for 8 hours. Plasmids were recovered from these bacterial cells by an alkali method and determined whether they were clones into which a desired fragment was cloned with the restriction enzyme, thereby giving plasmids pER23-1 and pER23-2 (5225 bp) which had opposite orientations to the vector. The maps of plasmids pER23-1 and pER23-2 are shown in Figure 2.

### 5) Determination of the Nucleotide Sequence of the ER-encoding Clone

The DNA nucleotide sequences of plasmids pER23-1 and pER23-2 were determined in both orientations with a fluorescence sequencer by 1) using plasmids pER23-1 and pER23-2 digested by appropriate restriction enzymes, 2) using appropriate primers synthesized on the basis of the information about already determined nucleotide sequences, or 3) cleaving pER23-1 with restriction enzymes KpnI and XhoI and pER23-2 with restriction enzymes KpnI and ClaI and then using a kilosequence deletion kit (TAKARA SHUZO CO., LTD) to prepare plasmids having a deletion at intervals of about 200-300 bp. The DNA nucleotide sequence is shown in SEQ ID NO: 2 of the SEQUENCE LISTING. The results showed that the DNA fragment contained a 667 amino acid-encoding open reading frame of 2001 bp beginning at a nucleotide sequence corresponding to the N-terminal sequence (fragmented peptide #1) sequenced with the amino acid sequencer. The amino acid sequence is shown in SEQ ID NO: 1 of the SEQUENCE LISTING. The amino acid sequence deduced from the resulting DNA nucleotide sequence was consistent with the previously determined amino acid sequence of the soybean ER.

In addition, highly homologous amino acid sequences were searched for with a nucleic acid and amino acid sequence analysis software package (MacVector: Kodak Co.) using a nucleic acid and amino acid sequence data base (Entrez: NCBI). However, no amino acid sequences were found to be highly homologous to the heretofore known sequences. Hence, it is clear that the prepared ER is a novel protein.

### Example 3

### Expression of the Soybean ER in Tobacco Plants

### 1) Construction of Plant Expression Plasmid pKV1-ER23

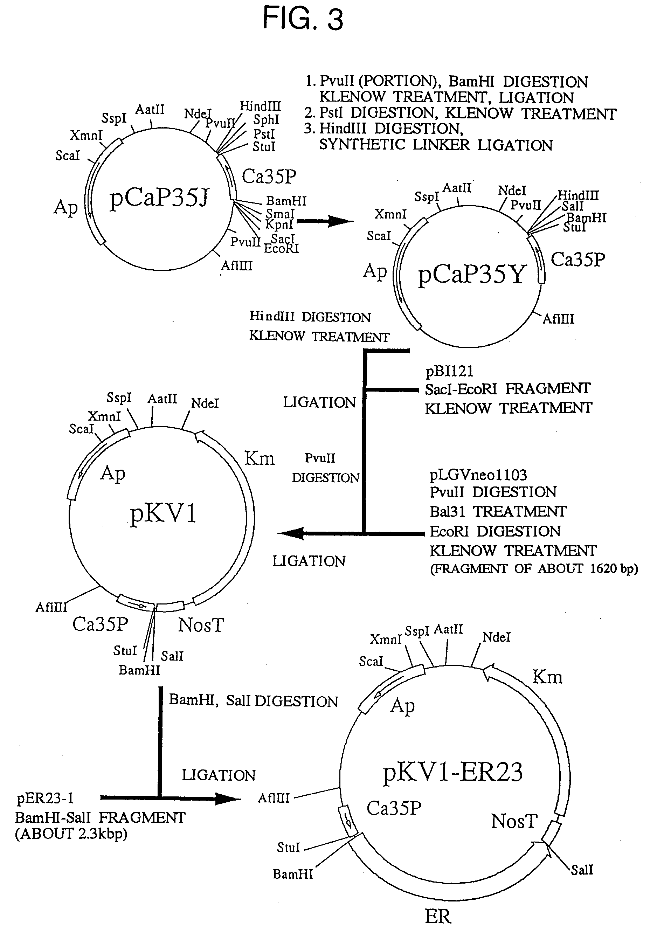

As shown in Figure 3, a plant expression vector pKV1 to be used in this example was prepared from cauliflower mosaic virus 35S promoter-containing plasmid pCaP35J (J. Yamaya et al. (1988) Mol. Gen. Genet. 211:520) as follows:

Plasmid pCaP35J was digested completely with restriction enzyme BamHI to delete a multi-cloning site present upstream of the 35S promoter. Following partial digestion with PvuII, a treatment was conducted with Klenow fragments (TAKARA SHUZO CO., LTD) to make blunt ends. The resulting plasmid DNA was circularized by ligation and introduced into E. coli DH5α. A desired plasmid was selected from the resulting clones. The selected plasmid was digested with restriction enzyme PstI to insert a multi-cloning site present downstream of the 35S promoter. A treatment was conducted with Klenow fragments to make blunt ends. The resulting plasmid DNA was digested with HindIII. The following synthetic linker DNAs were synthesized with an automatic nucleic acid synthesizer, annealed and ligated to the HindIII-digested plasmid. The resulting plasmid DNA was introduced into E. coli DH5α. Desired plasmid pCaP35Y (2837 bp) was selected from the obtained clones.

5'-GGAATTCGAGCTCGGTACCCGGGGATCCTCTAGAGTCGACCTGCAGGCATGCA-3'(SEQ ID NO:
3)
5'-CCTTAAGCTCGAGCCATGGGCCCCTAGGAGATCTCAGCTGGACGTCCGTACGTTCGA-3'(SEQ ID
NO:4)

In order to introduce a terminator of nopaline synthase into the pCaP35Y, plasmid pBI121 (Clontech Co.) was digested with SacI and EcoRI and the SacI-EcoRI fragment was treated with Klenow fragments to make blunt ends; then, the resulting fragment of pBI121 was ligated to plasmid pCaP35Y in which blunt ends were made at a HindIII site downstream of the 35S promoter. The resulting plasmid DNA was introduced into E. coli DH5α. A desired plasmid was selected from the obtained clones. In order to introduce a kanamycin-resistance cassette into the selected plasmid, the latter was digested with PvuII and ligated to a fragment (about 1620 bp) of pLGVneo1103 (R. Hain et al. (1985) Mol. Gen. Genet. 199: 161) that was obtained by the steps of cleavage at a PvuII site present downstream of the octopine synthase terminator, treatment with Bal31 (TAKARA SHUZO CO., LTD) to make a deletion, cleavage at a EcoRI site upstream of a nopaline synthase promoter, and the creation of a blunt end at both ends. The resulting plasmid DNA was introduced into E. coli DH5α. Desired plasmid, or plant expression vector pKV1 (4828 bp), was selected from the obtained clones.

The prepared pKV1 was digested at unique sites by restriction enzymes BamHI and SalI and ligated to the ER gene-containing fragment (i.e., the BamHI-SalI fragment of pEB23-1, about 2.3 kbp). The resulting plasmid DNA was introduced into E. coli DH5α. Desired ER-expression plasmid pKV1-ER23 (about 7.1 kbp) was selected from the obtained clones.

### 2) Transient Expression of ER in Cultured Tobacco Cells

The ER gene was introduced into cultured tobacco cells by electroporation for transient ER expression by a partial modification of Watanabe's method (Y. Watanabe (1987) FEBS 219: 65). The DNA molecules of the plasmid pKV1-ER23 were purified by an alkali method. Cultured tobacco cells were obtained by the method of Hirai et al. (Plant Cell Cultivation Manual, Gakkai Shuppan Center, 1982) for use in the transient expression of ER. Tobacco seeds (variety Bright Yellow, provided by Professor Hirofumi Uchimiya of University of Tokyo) were sterilized with a 1% sodium hypochlorite solution and then germinated. The tobacco juvenile tissues just after the germination were transplanted in a tobacco cultivation agar medium (Murashige-Skoog medium (Flow Laboratories Co.) supplemented with 2 ppm 2,4-dichlorophenoxyacetic acid, 3% sucrose and 8% agar) to induce calluses after 3 weeks. About 1 g of callus masses were suspended in 50 ml of a tobacco cultivation medium (Murashige-Skoog medium (Flow Laboratories Co.) supplemented with 2 ppm 2,4-dichlorophenoxyacetic acid and 3% sucrose) to prepare cultured cells. These tobacco cells were cultured until they entered a logarithmic growth phase. The cultured cells were collected by centrifugation (600 rpm, 3 minutes) and suspended in a solution consisting of 1% cellulase Onozuka (Yakult Co.), 1% Dricelase (Kyowa Hakko Co., Ltd.), 0.1% Pectriase (Seishin Seiyaku Co.) and 0.4 M D-mannitol (Wako Pure Chemicals Co., Ltd.) and which was adjusted to pH 5.7 with HCl. Reaction was performed at 30 °C for 90 minutes to prepare protoplasts. The reaction solution was washed with 0.4 M D-mannitol at 4 °C by 3 cycles of centrifugation to remove the enzyme solution. The operation of electroporation consisted of suspending 1 x 10⁶ cells in 0.8 ml of an electroporation solution (70 mM KCl, 5 mM MES and 0.3 M mannitol), mixing the suspension with 10 µg of the DNA molecules of pKV1-ER23 and treating the mixture with a genepluser (Biorad Co.) at 125 µF and 300 V in an electroporation cuvette (Biorad Co., electrode spacing: 0.4 cm). After the treatment, the solution was collected with a pasteur pipet and left to stand on ice for 30 minutes. Reaction was performed at 30 °C for 5 minutes and the reaction solution was resuspended in a protoplast medium (Murashige-Skoog medium (Flow Laboratories Co.) supplemented with 0.2 ppm 2,4-dichlorophenoxyacetic acid, 1% sucrose and 0.4 M mannitol and adjusted to pH 5.7). The cells were left to stand in the dark at 25°C overnight and collected by centrifugation (8,000 rpm, 3 minutes). Sixty microliters of a suspension buffer (25 mM Tris-HCl pH7.0, 30 mM MgCl₂, 2 mM dithiothreitol, 2.5 mM potassium metabisulfite and 1 mM PMSF) were added to the cells and the mixture was stirred on a vortex for 3 minutes. The resulting sample was stored at -80°C until an elicitor-binding experiment was conducted.

For control, the above procedure was repeated except that the DNA molecule of pKV1 instead of pKV1-ER23 was introduced into tobacco cells.

### 3) Stable Expression of ER in Tobacco Suspension Cultured Cells

Transformed cultured tobacco cells capable of constant ER gene retention were selected as follows from the cultured tobacco cells capable of transient ER expression:

The protoplasts obtained in the preparation of the cultured tobacco cells capable of transient ER expression were suspended in a 1% agarose-containing protoplast medium (Murashige-Skoog medium (Flow Laboratories Co.) supplemented with 0.2 ppm 2,4-dichlorophenoxyacetic acid, 1% sucrose and 0.4 M mannitol and adjusted to pH 5.7). The suspension was dropped on a plate with a dropping pipet before the agarose solidified, whereby the protoplasts were fixed in the bead-like solid medium. After the agarose solidified, an agarose-free protoplast medium was added to the plate, thereby immersing the protoplast-fixing agarose medium in the liquid medium. After the protoplasts were cultured in the dark for 1 week, kanamycin was added to a final concentration of 100 µg/ml and the cultivation was continued. Transformants selected from the grown colonies were transferred in a kanamycin-containing liquid medium and cultured.

Two clones (I 1 and I 6) of cultured tobacco cells stably transformed by pKV1-ER23 and two clones (C 2-1 and C 2-4) of cultured tobacco cells stably transformed by pKV1 were obtained.

### 4) Elicitor-binding Activity Experiment

The elicitor-binding activity was measured as follows:

A complex of an elicitor and tyramine (TOKYO KASEI KOGYO CO., LTD.) was synthesized by the method of Jong-Joo Cheong (The Plant Cell (1991) 3: 127). The elicitor-tyramine complex was labelled with iodine-125 using chloramine T. The resulting sample (protein amount < 500 µg) was suspended in 500 µl of an assay buffer (50 mM Tris-HCl pH7.4, 0.1 M saccharose, 5 mM MgCl₂, 1mM PMSF and 5 mM EDTA) and incubated at 0°C for 2 hours. The iodine-labelled elicitor-tyramine complex in an amount of 100 nM (70 Ci/mmol) was added to the suspension and the mixture was incubated at 4 °C for 2 hours. The reaction solution was filtered through Whatman GF/B (as treated with a 0.3% aqueous solution of polyethylenimine for at least 1 hour) and washed 3 times with 5 ml of an ice-cold buffer (10 mM Tris-HCl pH 7.0, 1 M NaCl, 10 mM MgCl₂). The radio activity retained on the filter membrane was counted with a gamma counter (count A). In order to eliminate the effect of non-specific binding, the same procedure as above was performed except that 17 µM of the elicitor was added to the same sample, the mixture was suspended in the assay buffer and the suspension was incubated at 0 °C for 2 hours. The obtained count was subtracted from the count A to give a count (Δ cpm) of elicitor-specific binding. The resulting count (Δ cpm) was divided by the total number of counts and then multiplied by the total amount of elicitor used in the experiment to calculate the amount of the elicitor-binding protein (in moles).

As a result, a specific binding to the elicitor was observed in the tobacco cells transformed with the DNA molecule of pKV1-ER23, whereas no specific binding to the elicitor was observed in the control tobacco cells in which the DNA molecule of pKV1 was introduced (Table 2). This fact reveals that the gene obtained above encodes a protein having the elicitor-binding activity.

**Table 2**

| Elicitor-binding Activity of Cultured Tobacco Cells | | |
|---|---|---|
| Fraction | Transfoming DNA | Binding Activity (fmol/mg) |
| Transient Expression | pKV1 | < 0 |
| | pKV1-ER23 | 90.5 |

| Stable Expression | | |
|---|---|---|
| C2-1 | pKV1 | < 0 |
| C2-4 | pKV1 | < 0 |
| I 1 | pKV1-ER23 | 150 |
| I 6 | pKV1-ER23 | 196 |

### 5) Transient Increase in Intracellular Ca²⁺ Concentration in Transformed Tobacco Cultured Cells by Addition of Glucan Elicitor

Plants recognize the elicitor at a receptor specific thereto and then promote the accumulation of phytoalexin or induce hypersensitive reaction to prevent fungus invasion. It has been reported for some plants that the inflow of calcium ion into cells in the early phase of such resistance reactions is important (U. Conrath et al. (1991) FEBS LETTERS 279: 141, M. N. Zook et al. (1987) Plant Physiol. 84: 520, F. Kurosaki et al. (1987) Phytochemistry 26: 1919; C. L. Preisig and R. A. Moreau (1994) Phytochemistry 36: 857). A report has also been made suggesting that the inflow of calcium ion into cells triggers the promotion of the phytoalexin accumulation in soybean which the present inventors used to obtain ER (M. R. Stab and J. Ebel (1987) Archi. Biochem. Biophys. 257: 416). Hence, if a transformed cultured tobacco cell is prepared by introducing the ER gene into an ER-free tobacco cultured cell to express the ER and if the intracellular calcium ion concentration is changed by the addition of a glucan elicitor, the change is anticipated to trigger a resistance reaction by the glucan elicitor in plants other than soybean (e.g., tobacco), thereby allowing them to show resistance to a wide variety of fungi which use glucan as a mycelial wall component.

The change in intracellular Ca²⁺ concentration of transformed cultured tobacco cells by the addition of the elicitor was examined.

In this experiment, the transformed cultured tobacco cells (I 6) obtained by the kanamycin selection and the plasmid-containing cultured tobacco cells (C 2-4) were used.

The intracellular Ca²⁺ concentrations of the cultured cells were measured as follows with an acetoxymethyl derivative (Fura-2 AM) of a fluorescence chelator (Fura-2) for Ca²⁺ measurement:

Cells were harvested from about 2 ml of the transformed tobacco cell culture (corresponding to a cell volume of about 250 ml after standing for 10 minutes) by centrifugation (600 rpm, 30 seconds) and the supernatant was removed. To the cells was added 2 ml of a tobacco cultivation medium and the mixture was stirred gently and centrifuged (600 rpm, 30 seconds) to remove the supernatant. The same operations were repeated to wash the cultured cells. The washed cultured cells was suspended homogeneously in 2 ml of the medium. To 1 ml of the suspension of the cultured cells in the medium, 1 ml of the medium and 4 µl of 1 mM Fura-2 AM (final concentration: 2 µM, Dojin Chemical Co.) were added and the mixture was incubated in the dark for 30 minutes with occasionally stirring. Subsequently, the cells were washed 2 times with 2 ml of the medium by centrifugation (600 rpm, 30 seconds) to eliminate the free Fura-2 AM which was not incorporated into the cells. The washed cultured cells were suspended in 2 ml of the medium homogeneously and the suspension (2 ml) was transferred into a fluorescence-measurement cell. The incorporated Fura-2 AM should be changed to Fura-2 by hydrolysis with intracellular esterase. The fluorescence produced by the binding of Fura-2 to intracellular Ca²⁺ was measured at a fluorescence wavelength of 505 nm under exciting light of 335 nm with the cultured cells being stirred to ensure against precipitation of the cultured cells. The change in intracellular Ca²⁺ concentration was examined by measuring the fluorescence intensity at specified intervals of time after the addition of 50 µl of glucan elicitor (1 mg/ml) or deionized water to the cultured cells. For control, the change in intracellular Ca²⁺ concentration was examined on the plasmid-containing cultured tobacco cells by the same method as above. For another control, the change in intracellular Ca²⁺ concentration was examined on cultured soybean cells by the same method as above, except that the cultured cells were washed with a medium for soybean cells having the following formulation.
NaH₂PO₄·H₂O 75mg/ml, KH₂PO₄ 170mg/ml, KNO₃ 2,200mg/ml, NH₄NO₃ 600mg/ml, (NH₄)₂SO₄ 67mg/ml, MgSO₄·7H₂O 310mg/ml, CaCl₂·2H₂O 295mg/ml, FeSO₄·7H₂O 28mg/ml, EDTA·Na₂ 37.3mg/ml, KI 0.75mg/ml, MnSO₄·4H₂O 10.0mg/ml, H₃BO₃ 3.0mg/ml, ZnSO₄·7H₂O 2mg/ml, Na₂MoO₄·2H₂O 0.25mg/ml, CuSO₄·5H₂O 0.025mg/ml, CoCl₂·6H₂O 0.025mg/ml, Inositol 100mg/ml, Nicotinic acid 1.0mg/ml, Pyridoxine·HCl 1.0mg/ml, Thiamine·HCl 10.0mg/ml, Glucose 5g/ml, Sucrose 25g/ml, Xylose 250mg/ml, Sodium pyruvate 5.0mg/ml, Citric acid 10.0mg/ml, Malic acid 10.0mg/ml, Fumaric acid 10.0mg/ml, N-Z-amine 500.0mg/ml, 2,4-dichlorophenoxyacetic acid 1.0mg/ml and Zeatine riboside 0.1mg/ml, adjusted to pH 5.7 with KOH.

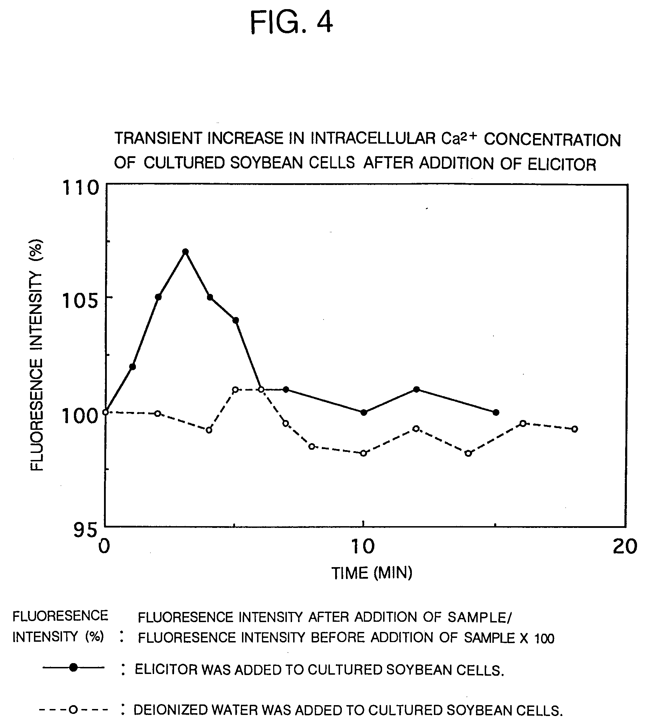

As a result of this experiment, about 7% transient increase in fluorescence intensity was observed in the cultured soybean cells 3 minutes after the addition of the elicitor, whereas no such change was observed after the addition of deionized water (Figure 4). The results suggest that the phenomenon in which the binding of the ER to the glucan elicitor caused a transient inflow of Ca²⁺ into cells could be observed in this experiment, thereby supporting the report that calcium ion plays an important role in the resistance reaction caused by the elicitor in cultured soybean cells. In the transformed cultured tobacco cells, about 10% transient increase in fluorescence intensity was observed 3 minutes after the addition of the elicitor, whereas no such change was observed after the addition of deionized water.

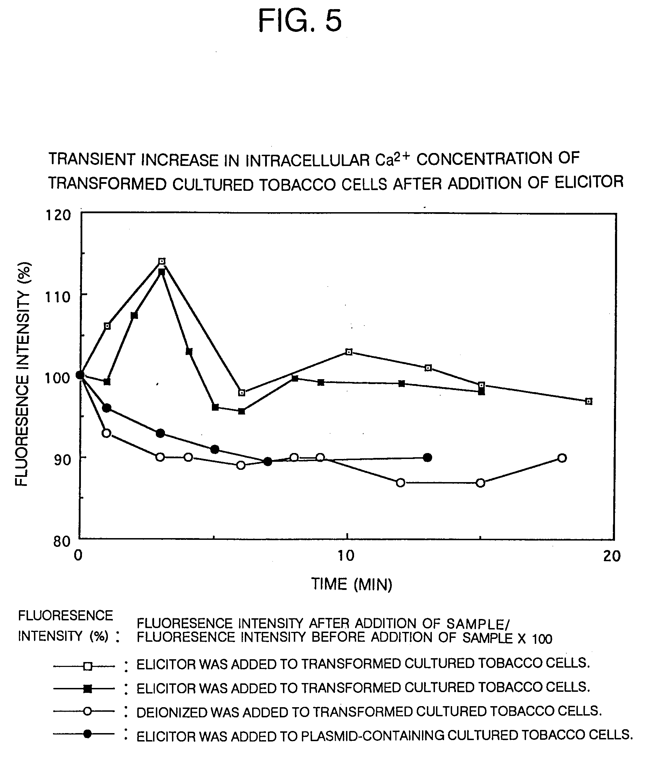

In the plasmid-containing cultured tobacco cells, none of the changes in fluorescence intensity that occurred in the transformed cultured tobacco cells was observed after the addition of the elicitor (Figure 5).

These results show that plants other than soybean (e.g., tobacco), which are not reactive with the glucan elicitor acquire the reactivity by introducing the gene of the soybean-derived glucan elicitor receptor for ER expression. Although the signal transduction pathway of each plant has not been completely explicated, it is expected that plants other than tobacco will acquire the reactivity with the glucan elicitor (i.e., a transient increase in intracellular Ca²⁺ concentration) by introducing the gene of the present ER for ER expression, thereby enabling the development of plants having resistance to a wide variety of fungi which use glucan as a mycelial wall component.

### Example 4

### Expression of Soybean ER in E. coli and Determination of Elicitor-binding Domain

### 1) Expression of Elicitor-binding Domain in E. coli

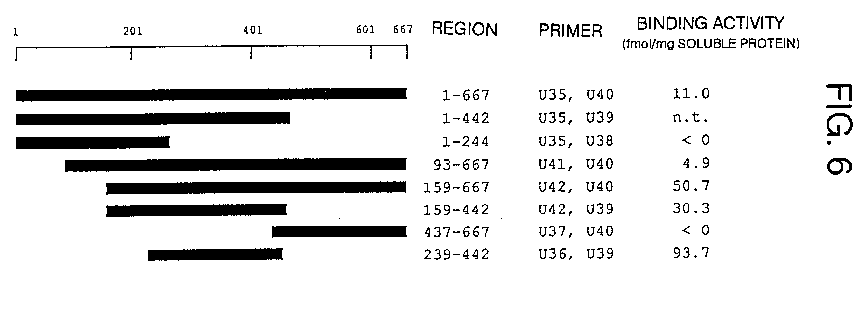

A fused protein of a partial fragment of the soybean ER with a maltose-binding protein (MBP) was prepared with a Protein Fusion & Purification System (New England Biolabs Co.) in order to express the partial fragment of the soybean ER in E. coli. PCR was performed using pER23-1 as a template to give DNA fragments of various lengths. The primers were designed to produce the MBP and fused protein in cloning into plasmid pMAL-c2 (New England Biolabs Co.) by adding a BamHI site on the 5' side and a SalI site on the 3' side exterior to the DNA molecule encoding the full-length portion and fragments of soybean ER shown in Figure 6. These primers were synthesized with an automatic nucleic acid synthesizer (Model 394 of Applied Biosystems Co.). The following primers were used in the amplification of the DNA chain.
- Primer U35: 5'-ATGGATCCATGGTTAACATCCAAACC-3';
- Primer U36: 5'-ATGGATCCGAATATAACTGGGAGAAG-3';
- Primer U37: 5'-ATGGATCCCCAGCATGGGGTAGGAAG-3';
- Primer U38: 5'-TAGTCGACTACTTCTCCCAGTTATATTC-3';
- Primer U39: 5'-TAGTCGACTACTTCCTACCCCATGCTGG-3';
- Primer U40: 5'-TAGTCGACTATTCATCACTTCTGCTATG-3';
- Primer U41: 5'-ATGGATCCGCCCCACAAGGTCCCAAA-3'; and
- Primer U42: 5'-ATGGATCCAATGACTCCAACACCAAG-3'

The DNA molecule of pER23-1 (0.01 µg) was dissolved in 79 µl of distilled water. Either a combination of primers U5 and U7 or a combination of primers U10 and U12 (100 pmol each) and 0.5 µl of Taq DNA polymerase (TAKARA SHUZO CO., LTD.) were added to 8 µl of 2.5 mM dNTP in 10 µl of a 10 x PCR buffer (attached to Taq DNA polymerase of TAKARA SHUZO CO., LTD.) to give a final amount of 100 µl. PCR reaction was performed with a Gene Amp PCR System 9600 (Perkin-Elmer Co.) by 30 cycles of 1) denaturation at 94 °C x 30 seconds, 2) renaturation at 55°C x 30 seconds and 3) elongation at 72 °C x 1 minute. After the reaction, 15 µl of the reaction solution was digested with restriction enzymes BamHI and SalI and electrophoresed on a 1% agarose gel.

The gel was stained with a 0.5 µg/ml ethidium bromide solution for 15 minutes. The band showing the expected specific amplification was sectioned while observing under UV light. The gel section was treated with Gene Clean II (Bio101 Co.) to collect a DNA-containing solution. The collected DNA fragments were cloned into the BamHI-SalI site of plasmid pMAL-c2 and the clones were introduced into E. coli DH5α.

### 2) Preparation of Soluble Protein Fraction from E. coli

The E. coli cells into which the plasmids were introduced were precultured in an expression medium [10g/l tryptone (Gibco Co.), 5 g/l yeast extract (Gibco Co.), 5 g/l NaCl, 2 g/l glucose and 100 µg/ml ampicillin]. The precultured solution (0.4 ml) was added to 40 ml of the expression medium and cultured at 37 °C with shaking until OD₆₀₀ of 0.55 was reached. Isopropylthiogalactoside was added to the culture solution to give a final concentration of 0.3 mM and the shakeculture was continued for an additional 4 hours to induce expression. The E. coli was collected by centrifugation and the E. coli cells were washed with a washing buffer (20 mM Tris-HCl, pH 7.4, 200 mM NaCl and 1 mM EDTA). The cells were sonicated for a total of 2 minutes (15 sec x 8). ZW3-12 was added to the sonicated cells to give a final concentration of 0.25% and the mixture was incubated at 4 °C for 30 minutes. The supernatant was collected by centrifugation (10,000 rpm, 5 minutes) to give an E. coli soluble protein fraction. The expression of the fused protein was confirmed by an immunoblotting technique using an anti-maltose-binding protein antibody (New England Biolabs Co.).

### 3) Elicitor-binding Experiment

The elicitor-binding activity was determined as follows:

A complex of an elicitor and tyramine (TOKYO KASEI KOGYO CO., LTD.) was synthesized by the method of Jong-Joo Cheong (The Plant Cell (1991) 3: 127). The elicitor-tyramine complex was labelled with iodine-125 using chloramine T. The resulting sample (protein amount < 800 µg) was suspended in 500 µl of an assay buffer (50 mM Tris-HCl pH7.4, 0.1 M saccharose, 5 mM MgCl₂, 1mM PMSF and 5 mM EDTA) and incubated at 0°C for 2 hours. The iodine-labelled elicitor-tyramine complex in an amount of 100 nM (70 Ci/mmol) was added to the suspension and the mixture was incubated at 4 °C for 2 hours. The reaction solution was filtered through Whatman GF/B (as treated with a 0.3% aqueous solution of polyethylenimine for at least 1 hour) and washed 3 times with 5 ml of an ice-cold buffer (10 mM Tris-HCl pH 7.0, 1 M NaCl, 10 mM MgCl₂). The radio activity retained on the filter membrane was counted with a gamma counter (count A). In order to eliminate the effect of non-specific binding, the same procedure as above was performed except that 17 µM of the elicitor was added to the same sample, the mixture was suspended in the assay buffer and the suspension was incubated at 0 °C for 2 hours. The obtained count was subtracted from the count A to give a count (Δ cpm) of elicitor-specific binding. The resulting count (Δ cpm) was divided by the total number of counts and then multiplied by the total amount of the elicitor used in the experiment to calculate the amount of the elicitor-binding protein (in moles).

As a result, a specific binding to the elicitor was observed in the E. coli transformed with the DNA molecule encoding the ER (Figure 6). Hence, it was reconfirmed that the obtained gene encoded a protein having the elicitor-binding activity and it was revealed that there was an elicitor-binding domain in the 239-442 amino acid sequence of SEQ ID NO:1.

### Example 5

### Inhibition of Binding of Glucan Elicitor to Elicitor-binding Protein in Soybean Cotyledon Membrane Fraction and Inhibition of Accumulation of Phytoalexin in Soybean Cotyledon by Antibody against Elicitor-binding Domain

### 1) Expression of Elicitor-binding Domain in E. coli

A fused protein of an elicitor-binding domain derived from the ER with a maltose-binding protein (MBP) was prepared with a Protein Fusion & Purification System (New England Biolabs Co.) in order to express a large amount of the elicitor-binding domain in E. coli. PCR was performed to produce a DNA molecule encoding the elicitor-binding domain. The following primers were synthesized with an automatic nucleic acid synthesizer (Model 394 of Applied Biosystems Co.):
- Primer U36: 5'-ATGGATCCGAATATAACTGGGAGAAG-3'; and
- Primer U39: 5'-TAGTCGACTACTTCCTACCCCATGCTGG-3'

The DNA molecule of pER23-1 (0.01 µg) was dissolved in 79 µl of distilled water. Either a combination of primers U5 and U7 or a combination of primers U10 and U12 (100 pmol each) and 0.5 µl of Taq DNA polymerase (TAKARA SHUZO CO., LTD.) were added to 8 µl of 2.5 mM dNTP in 10 µl of a 10 x PCR buffer (attached to Taq DNA polymerase of TAKARA SHUZO CO., LTD.) to give a final amount of 100 µl. PCR was performed with a Gene Amp PCR System 9600 (Perkin-Elmer Co.) by 30 cycles of 1) denaturation at 94°C x 30 seconds, 2) renaturation at 55°C x 30 seconds and 3) elongation at 72 °C x 1 minute. After the reaction, 15 µl of the reaction solution was digested with restriction enzymes BamHI and SalI and electrophoresed on a 1% agarose gel.

The gel was stained with a 0.5 µg/ml ethidium bromide solution for 15 minutes. The band showing specific amplification was sectioned while observing under UV light. The gel section was treated with Gene Clean II (Bio101 Co.) to collect a DNA-containing solution. The collected DNA fragments were cloned into the BamHI-SalI site of plasmid pMAL-c2 and the clones were introduced into E. coli DH5α.

### 2) Purification of the Fused Protein Expressed in E. coli and Production of Antibody

The E. coli cells transformed with the plasmids were precultured in an expression medium (10g/l tryptone (Gibco Co.), 5 g/l yeast extract (Gibco Co.), 5 g/l NaCl, 2 g/l glucose and 100 µg/ml ampicillin) overnight. The precultured solution (150 ml) was added to 1.5 L of the expression medium and cultured in a Sakaguchi flask at 37°C with shaking until OD₆₀₀ of 0.55 was reached. Isopropylthiogalactoside was added to the culture solution to give a final concentration of 0.3 mM and the shakeculture was continued for an additional 4 hours to induce expression. The E. coli was collected by centrifugation and the E. coli cells were washed with a washing buffer (20 mM Tris-HCl, pH 7.4, 200 mM NaCl and 1 mM EDTA). The cells were sonicated for a total of 2 minutes (15 sec x 8). A soluble protein fraction was obtained by centrifugation. From this fraction, a MBP-fused protein was purified with an amylose resin. A MBP- and an elicitor-binding domain were cleaved with factor Xa and the elicitor-binding domain was purified by gel filtration column chromatography. The purified protein was injected twice into a mouse at the abdominal cavity for immunization by the method of E. Harlow and D. Lane (Antibody (1988) Cold Spring Harbor Co., pp. 53-137). After the increase in titer was confirmed by an ELISA method, the ascites was obtained and subjected to precipitation with 50% saturated ammonium sulfate and treatment with Protein A Sepharose (Pharmacia Co.) to produce a purified antibody. In the treatment with Protein A Sepharose, the antibody was bound to Protein A Sepharose with 0.1 M sodium phosphate (pH 8.0) and eluted with 0.1 M citric acid (pH 3.5). It was confirmed by an immnoblotting that the obtained antibody recognized only the ER protein in soybean.

### 3) Preparation of Soybean Cotyledon Membrane Fraction

A soybean cytoledon membrane fraction was prepared as follows:

Soybean seeds were cultured on soil for 9 days and the cytoledons were harvested (36 g, wet weight). The cytoledons were suspended in 47 ml of an ice-cold buffer (25 mM Tris-HCl pH 7.0, 30 mM MgCl₂, 2 mM dithiothreitol, 2.5 mM sodium metabisulfite and 1 mM PMSF). A cytoledon membrane fraction was prepared by the same method as the one for preparing the membrane fraction from the soybean roots. The resulting cytoledon membrane fraction was suspended in 1 ml of an ice-cold buffer (10 mM Tris-HCl pH 7.4, 0.1 M sucrose, 5 mM MgCl₂, 1 mM PMSF and 5 mM EDTA) and stored at -80 °C.

### 4) Measurement of Inhibition of Glucan Elicitor Binding to Elicitor-binding Protein of Soybean Cotyledon Membrane Fraction

The elicitor-binding activity was determined as follows:

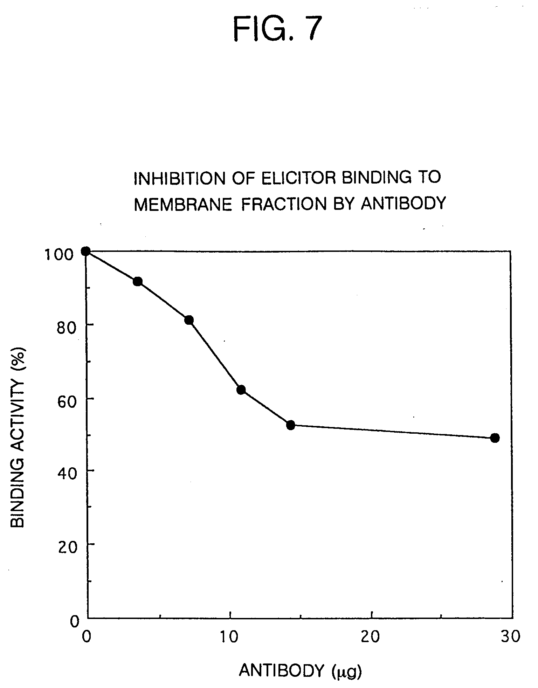

A complex of an elicitor and tyramine (TOKYO KASEI KOGYO CO., LTD.) was synthesized by the method of Jong-Joo Cheong (The Plant Cell (1991) 3: 127). The elicitor-tyramine complex was labelled with iodine-125 using chloramine T. The soybean cotyledon membrane faction (100 µl, 820 µg) was suspended in 500 µl of an assay buffer (50 mM Tris-HCl pH7.4, 0.1 M saccharose, 5 mM MgCl₂, 1mM PMSF and 5 mM EDTA) and incubated at 0°C for 2 hours. The iodine-labelled elicitor-tyramine complex in an amount of 714 ng (143 nM; 70 Ci/mmol) was added to the suspension and the mixture was incubated at 4 °C for 2 hours. The reaction solution was filtered through Whatman GF/B (as treated with a 0.3% aqueous solution of polyethylenimine for at least 1 hour) and washed 3 times with 5 ml of an ice-cold buffer (10 mM Tris-HCl pH 7.0, 1 M NaCl, 10 mM MgCl₂). The radio activity retained on the filter membrane was counted with a gamma counter (count A). In order to eliminate the effect of non-specific binding, the same procedure as above was performed, except that 100 times mole (75 µg, 15 µM) of a cold elicitor was added to the the sample, the mixture was suspended in the assay buffer and the suspension was incubated at 0°C for 2 hours. The obtained count was subtracted from the count A to give a count (Δ cpm) of elicitor-specific binding. Counts of binding obtained by adding 3.6, 7.1, 10.8, 14.4 and 28.8 µg of the purified antibody rather than the cold elicitor were subtracted from the count A. The resulting values were compared with that for the cold elicitor and expressed as the percentage, with the count (Δ cpm) of elicitor-specific binding being taken as 100% (Figure 7). The addition of 28.8 µg of the antibody resulted in the inhibition of the binding of elicitor by about 51%. The results confirmed that the antibody against the elicitor-binding domain inhibited the binding of the elicitor to the elicitor-binding protein.

### 5) Inhibition of Accumulation of Phytoalexin by Antibody against Elicitor-binding Domain

The amount of phytoalexin accumulated by the action of glucan elicitor was measured with soybean cotyledons by the method of M.G. Hahn et al. ((1992) Molecular Plant Pathology Volume II A Practical Approach, IRL Press, pp. 117-120).

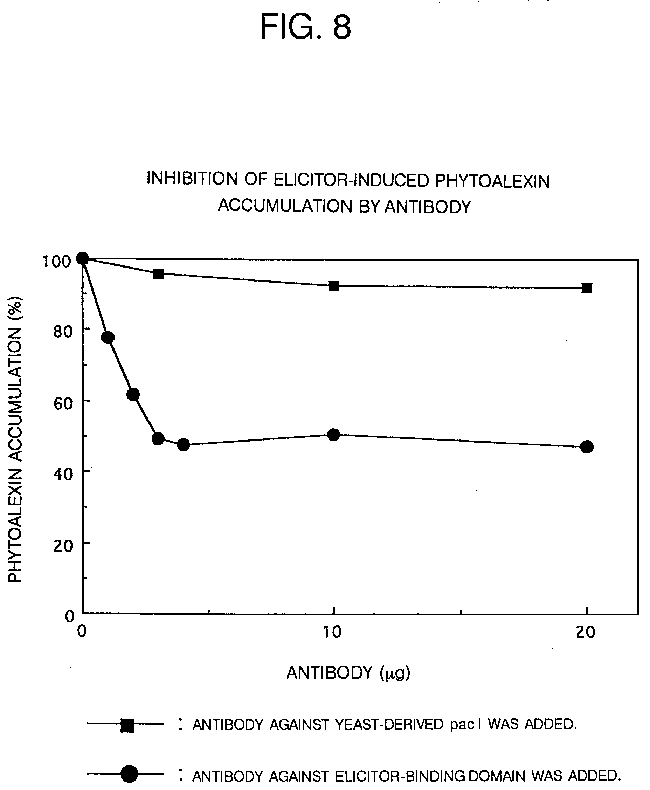

A purified antibody against the elicitor-binding domain (0, 1, 2, 3, 4, 10 and 20 µg/25 µl/cotyledon) or a purified antibody against yeast-derived dsRNAse, pac 1 (4, 10 and 20 µg/25 µl/cotyledon) as a control was added to soybean cotyledons and the mixture was incubated for 1 hour. Glucan elicitor (200 ng/25 µl/cotyledon) was added to the soybean cotyledons and the mixture was incubated for 20 hours to determine whether the accumulation of phytoalexin by the action of glucan elicitor was inhibited by the antibody. The amount of phytoalexin accumulation induced by the addition of elicitor subsequent to the addition of the antibody was expressed as the percentage, with the amount of phytoalexin accumulation by the sole addition of the elicitor being taken as 100% (Figure 8). When the antibody against the elicitor-binding domain was added in an amount of 20.0 µg per soybean cotyledon, the amount of phytoalexin accumulation decreased by about 53%. In the control, the amount of phytoalexin accumulation changed little even when the antibody against pac 1 was added in an amount of 20.0 µg per soybean cotyledon. These results showed that the obtained gene did not encode a mere elicitor-binding protein but encoded the ER inducing a resistance reaction in soybean.

### INDUSTRIAL APPLICABILITY

According to the present invention, a glucan elicitor receptor, DNA molecules coding for the glucan elicitor receptor and fragments thereof, vectors containing the DNA molecules or fragments thereof, and plant cells transformed with the DNA molecules or fragments thereof are provided.

The ER of the present invention is useful in the elucidation of resistance to fungi and the development of elicitor derivatives capable of inducing fungal resistance, and it can be used as an antigen for the production of antibodies against ERs.

The DNA molecules of the present invention which contain nucleotide sequences coding for the glucan elicitor receptor and fragments thereof are useful as materials for establishing techniques for developing fungi-resistant plants. In other words, the DNA molecules of the present invention and fragments thereof may be introduced and expressed in various plants to enhance their fungal resistance.

Antibodies against the glucan elicitor receptor of the present invention, the DNA molecules of the present invention which contain nucleotide sequences coding for the glucan elicitor receptor, their mutants and anti-sense DNAs can be used in the studies of the elicitor-binding site of ER and signal transduction.

Further, the information on the amino acid sequence of ER and the nucleotide sequence coding therefor can be used in the studies of the elicitor-binding site of ER and the signal transduction in which ER is involved.

Sequence Listing
INFORMATION FOR SEQ ID NO:1
LENGTH: 667 amino acids
TYPE: amino acid
TOPOLOGY: linear
MOLECULE TYPE: peptide
SEQUENCE DESCRIPTION: SEQ ID NO:1:
Val Asn Ile Gln Thr Asn Thr Ser Tyr Ile Phe Pro Gln Thr Gln Ser Thr Val
1               5                   10                  15
Leu Pro Asp Pro Ser Lys Phe Phe Ser Ser Asn Leu Leu Ser Ser Pro Leu Pro
    20                  25                  30                  35
Thr Asn Ser Phe Phe Gln Asn Phe Val Leu Lys Asn Gly Asp Gln Gln Glu Tyr
            40                  45                  50
Ile His Pro Tyr Leu Ile Lys Ser Ser Asn Ser Ser Leu Ser Leu Ser Tyr Pro
55                  60                  65                  70
Ser Arg Gln Ala Ser Ser Ala Val Ile Phe Gln Val Phe Asn Pro Asp Leu Thr
        75                  80                  85                  90
Ile Ser Ala Pro Gln Gly Pro Lys Gln Gly Pro Pro Gly Lys His Leu Ile Ser
                95                  100                 105
Ser Tyr Ser Asp Leu Ser Val Thr Leu Asp Phe Pro Ser Ser Asn Leu Ser Phe
    110                 115                 120                 125
Phe Leu Val Arg Gly Ser Pro Tyr Leu Thr Val Ser Val Thr Gln Pro Thr Pro
            130                 135                 140
Leu Ser Ile Thr Thr Ile His Ser Ile Leu Ser Phe Ser Ser Asn Asp Ser Asn
145                 150                 155                 160
Thr Lys Tyr Thr Phe Gln Phe Asn Asn Gly Gln Thr Trp Leu Leu Tyr Ala Thr
        165                 170                 175                 180
Ser Pro Ile Lys Leu Asn His Thr Leu Ser Glu Ile Thr Ser Asn Ala Phe Ser
                185                 190                 195

Gly Ile Ile Arg Ile Ala Leu Leu Pro Asp Ser Asp Ser Lys His Glu Ala Val
    200                 205                 210                 215
Leu Asp Lys Tyr Ser Ser Cys Tyr Pro Val Ser Gly Lys Ala Val Phe Arg Glu
            220                 225                 230
Pro Phe Cys Val Glu Tyr Asn Trp Glu Lys Lys Asp Ser Gly Asp Leu Leu Leu
235                 240                 245                 250
Leu Ala His Pro Leu His Val Gln Leu Leu Arg Asn Gly Asp Asn Asp Val Lys
        255                 260                 265                 270
Ile Leu Glu Asp Leu Lys Tyr Lys Ser Ile Asp Gly Asp Leu Val Gly Val Val
                275                 280                 285
Gly Asp Ser Trp Val Leu Lys Thr Asp Pro Leu Phe Val Thr Trp His Ser Ile
    290                 295                 300                 305
Lys Gly Ile Lys Glu Glu Ser His Asp Glu Ile Val Ser Ala Leu Ser Lys Asp
            310                 315                 320
Val Glu Ser Leu Asp Ser Ser Ser Ile Thr Thr Thr Glu Ser Tyr Phe Tyr Gly
325                 330                 335                 340
Lys Leu Ile Ala Arg Ala Ala Arg Leu Val Leu Ile Ala Glu Glu Leu Asn Tyr
        345                 350                 355                 360
Pro Asp Val Ile Pro Lys Val Arg Asn Phe Leu Lys Glu Thr Ile Glu Pro Trp
                365                 370                 375
Leu Glu Gly Thr Phe Ser Gly Asn Gly Phe Leu His Asp Glu Lys Trp Gly Gly
    380                 385                 390                 395
Ile Ile Thr Gln Lys Gly Ser Thr Asp Ala Gly Gly Asp Phe Gly Phe Gly Ile
            400                 405                 410
Tyr Asn Asp His His Tyr His Leu Gly Tyr Phe Ile Tyr Gly Ile Ala Val Leu
415                 420                 425                 430
Thr Lys Leu Asp Pro Ala Trp Gly Arg Lys Tyr Lys Pro Gln Ala Tyr Ser Ile
        435                 440                 445                 450
Val Gln Asp Phe Leu Asn Leu Asp Thr Lys Leu Asn Ser Asn Tyr Thr Arg Leu

                455                 460                 465

Arg Cys Phe Asp Pro Tyr Val Leu His Ser Trp Ala Gly Gly Leu Thr Glu Phe

    470                 475                 480                 485

Thr Asp Gly Arg Asn Gln Glu Ser Thr Ser Glu Ala Val Ser Ala Tyr Tyr Ser

            490                 495                 500

Ala Ala Leu Met Gly Leu Ala Tyr Gly Asp Ala Pro Leu Val Ala Leu Gly Ser

505                 510                 515                 520

Thr Leu Thr Ala Leu Glu Ile Glu Gly Thr Lys Met Trp Trp His Val Lys Glu

        525                 530                 535                 540

Gly Gly Thr Leu Tyr Glu Lys Glu Phe Thr Gln Glu Asn Arg Val Met Gly Val

                545                 550                 555

Leu Trp Ser Asn Lys Arg Asp Thr Gly Leu Trp Phe Ala Pro Ala Glu Trp Lys

    560                 565                 570                 575

Glu Cys Arg Leu Gly Ile Gln Leu Leu Pro Leu Ala Pro Ile Ser Glu Ala Ile

            580                 585                 590

Phe Ser Asn Val Asp Phe Val Lys Glu Leu Val Glu Trp Thr Leu Pro Ala Leu

595                 600                 605                 610

Asp Arg Glu Gly Gly Val Gly Glu Gly Trp Lys Gly Phe Val Tyr Ala Leu Glu

        615                 620                 625                 630

Gly Val Tyr Asp Asn Glu Ser Ala Leu Gln Lys Ile Arg Asn Leu Lys Gly Phe

                635                 640                 645

Asp Gly Gly Asn Ser Leu Thr Asn Leu Leu Trp Trp Ile His Ser Arg Ser Asp

    650                 655                 660                 665

Glu

667

INFORMATION FOR SEQ ID NO: 2

LENGTH: 2004 base pairs

TYPE: nucleic acid

STRANDEDNESS: double

TOPOLOGY: linear

MOLECULE TYPE: cDNA

ORIGINAL SOURCE:

ORGANISM: Soybean (Glycine max L.)

STRAIN: Green Homer

SEQUENCE DESCRIPTION: SEQ ID NO: 2:

9 18 27 36 45 54

GTT AAC ATC CAA ACC AAT ACA TCT TAC ATC TTC CCT CAA ACA CAA TCC ACT GTT

Val Asn Ile Gln Thr Asn Thr Ser Tyr Ile Phe Pro Gln Thr Gln Ser Thr Val

63 72 81 90 99 108

CTT CCT GAT CCC TCC AAA TTC TTC TCC TCA AAC CTT CTC TCA AGT CCA CTC CCC

Leu Pro Asp Pro Ser Lys Phe Phe Ser Ser Asn Leu Leu Ser Ser Pro Leu Pro

117 126 135 144 153 162

ACA AAC TCT TTC TTC CAA AAC TTT GTC CTA AAA AAT GGT GAC CAA CAA GAA TAC

Thr Asn Ser Phe Phe Gln Asn Phe Val Leu Lys Asn Gly Asp Gln Gln Glu Tyr

171 180 189 198 207 216

ATT CAT CCT TAC CTC ATC AAA TCC TCC AAC TCT TCC CTC TCT CTC TCA TAC CCT

Ile His Pro Tyr Leu Ile Lys Ser Ser Asn Ser Ser Leu Ser Leu Ser Tyr Pro

225 234 243 252 261 270

TCT CGC CAA GCC AGT TCA GCT GTC ATA TTC CAA GTC TTC AAT CCT GAT CTT ACC

Ser Arg Gln Ala Ser Ser Ala Val Ile Phe Gln Val Phe Asn Pro Asp Leu Thr

279 288 297 306 315 324

ATT TCA GCC CCA CAA GGT CCC AAA CAA GGT CCC CCT GGT AAA CAC CTT ATC TCC

Ile Ser Ala Pro Gln Gly Pro Lys Gln Gly Pro Pro Gly Lys His Leu Ile Ser

333 342 351 360 369 378
TCC TAC AGT GAT CTC AGT GTC ACC TTG GAT TTC CCT TCT TCC AAT CTG AGC TTC
Ser Tyr Ser Asp Leu Ser Val Thr Leu Asp Phe Pro Ser Ser Asn Leu Ser Phe

387 396 405 414 423 432
TTC CTT GTT AGG GGA AGC CCC TAT TTG ACT GTG TCT GTG ACT CAA CCA ACT CCT
Phe Leu Val Arg Gly Ser Pro Tyr Leu Thr Val Ser Val Thr Gln Pro Thr Pro

441 450 459 468 477 486
CTT TCA ATT ACC ACC ATC CAT TCC ATT CTC TCA TTC TCT TCA AAT GAC TCC AAC
Leu Ser Ile Thr Thr Ile His Ser Ile Leu Ser Phe Ser Ser Asn Asp Ser Asn

495 504 513 522 531 540
ACC AAG TAC ACC TTT CAG TTC AAC AAT GGT CAA ACA TGG CTT CTT TAT GCT ACC
Thr Lys Tyr Thr Phe Gln Phe Asn Asn Gly Gln Thr Trp Leu Leu Tyr Ala Thr

549 558 567 576 585 594
TCC CCC ATC AAG TTG AAC CAC ACC CTT TCT GAG ATA ACT TCT AAT GCA TTT TCT
Ser Pro Ile Lys Leu Asn His Thr Leu Ser Glu Ile Thr Ser Asn Ala Phe Ser

603 612 621 630 639 648
GGC ATA ATC CGG ATA GCT TTG TTG CCG GAT TCG GAT TCG AAA CAC GAG GCT GTT
Gly Ile Ile Arg Ile Ala Leu Leu Pro Asp Ser Asp Ser Lys His Glu Ala Val

657 666 675 684 693 702
CTT GAC AAG TAT AGT TCT TGT TAC CCC GTG TCA GGT AAA GCT GTG TTC AGA GAA
Leu Asp Lys Tyr Ser Ser Cys Tyr Pro Val Ser Gly Lys Ala Val Phe Arg Glu

711 720 729 738 747 756
CCT TTC TGT GTG GAA TAT AAC TGG GAG AAG AAA GAT TCA GGG GAT TTG CTA CTC
Pro Phe Cys Val Glu Tyr Asn Trp Glu Lys Lys Asp Ser Gly Asp Leu Leu Leu

765 774 783 792 801 810
TTG GCT CAC CCT CTC CAT GTT CAG CTT CTT CGT AAT GGA GAC AAT GAT GTC AAA
Leu Ala His Pro Leu His Val Gln Leu Leu Arg Asn Gly Asp Asn Asp Val Lys

819 828 837 846 855 864
ATT CTT GAA GAT TTA AAG TAT AAA AGC ATT GAT GGG GAT CTT GTT GGT GTT GTC
Ile Leu Glu Asp Leu Lys Tyr Lys Ser Ile Asp Gly Asp Leu Val Gly Val Val

873 882 891 900 909 918
GGG GAT TCA TGG GTT TTG AAA ACA GAT CCT TTG TTT GTA ACA TGG CAT TCA ATC
Gly Asp Ser Trp Val Leu Lys Thr Asp Pro Leu Phe Val Thr Trp His Ser Ile

927 936 945 954 963 972
AAG GGA ATC AAA GAA GAA TCC CAT GAT GAG ATT GTC TCA GCC CTT TCT AAA GAT
Lys Gly Ile Lys Glu Glu Ser His Asp Glu Ile Val Ser Ala Leu Ser Lys Asp

981 990 999 1008 1017 1026
GTT GAG AGC CTA GAT TCA TCA TCA ATA ACT ACA ACA GAG TCA TAT TTT TAT GGG
Val Glu Ser Leu Asp Ser Ser Ser Ile Thr Thr Thr Glu Ser Tyr Phe Tyr Gly

1035 1044 1053 1062 1071 1080
AAG TTG ATT GCA AGG GCT GCA AGG TTG GTA TTG ATT GCT GAG GAG TTG AAC TAC
Lys Leu Ile Ala Arg Ala Ala Arg Leu Val Leu Ile Ala Glu Glu Leu Asn Tyr

1089 1098 1107 1116 1125 1134
CCT GAT GTG ATT CCA AAG GTT AGG AAT TTT TTG AAA GAA ACC ATT GAG CCA TGG
Pro Asp Val Ile Pro Lys Val Arg Asn Phe Leu Lys Glu Thr Ile Glu Pro Trp

1143 1152 1161 1170 1179 1188
TTG GAG GGA ACT TTT AGT GGG AAT GGA TTC CTA CAT GAT GAA AAA TGG GGT GGC
Leu Glu Gly Thr Phe Ser Gly Asn Gly Phe Leu His Asp Glu Lys Trp Gly Gly

1197 1206 1215 1224 1233 1242
ATT ATT ACC CAA AAG GGG TCC ACT GAT GCT GGT GGT GAT TTT GGA TTT GGA ATT
Ile Ile Thr Gln Lys Gly Ser Thr Asp Ala Gly Gly Asp Phe Gly Phe Gly Ile

1251 1260 1269 1278 1287 1296
TAC AAT GAT CAC CAC TAT CAT TTG GGG TAC TTC ATT TAT GGA ATT GCG GTG CTC
Tyr Asn Asp His His Tyr His Leu Gly Tyr Phe Ile Tyr Gly Ile Ala Val Leu

1305 1314 1323 1332 1341 1350
ACT AAG CTT GAT CCA GCA TGG GGT AGG AAG TAC AAG CCT CAA GCC TAT TCA ATA
Thr Lys Leu Asp Pro Ala Trp Gly Arg Lys Tyr Lys Pro Gln Ala Tyr Ser Ile

1359 1368 1377 1386 1395 1404
GTG CAA GAC TTC TTG AAC TTG GAC ACA AAA TTA AAC TCC AAT TAC ACA CGT TTG
Val Gln Asp Phe Leu Asn Leu Asp Thr Lys Leu Asn Ser Asn Tyr Thr Arg Leu

1413 1422 1431 1440 1449 1458
AGG TGT TTT GAC CCT TAT GTG CTT CAC TCT TGG GCT GGA GGG TTA ACT GAG TTC
Arg Cys Phe Asp Pro Tyr Val Leu His Ser Trp Ala Gly Gly Leu Thr Glu Phe

1467 1476 1485 1494 1503 1512

ACA GAT GGA AGG AAT CAA GAG AGC ACA AGT GAG GCT GTG AGT GCA TAT TAT TCT
Thr Asp Gly Arg Asn Gln Glu Ser Thr Ser Glu Ala Val Ser Ala Tyr Tyr Ser

1521 1530 1539 1548 1557 1566
GCT GCT TTG ATG GGA TTA GCA TAT GGT GAT GCA CCT CTT GTT GCA CTT GGA TCA
Ala Ala Leu Met Gly Leu Ala Tyr Gly Asp Ala Pro Leu Val Ala Leu Gly Ser

1575 1584 1593 1602 1611 1620
ACA CTC ACA GCA TTG GAA ATT GAA GGG ACT AAA ATG TGG TGG CAT GTG AAA GAG
Thr Leu Thr Ala Leu Glu Ile Glu Gly Thr Lys Met Trp Trp His Val Lys Glu

1629 1638 1647 1656 1665 1674
GGA GGT ACT TTG TAT GAG AAA GAG TTT ACA CAA GAG AAT AGG GTG ATG GGT GTT
Gly Gly Thr Leu Tyr Glu Lys Glu Phe Thr Gln Glu Asn Arg Val Met Gly Val

1683 1692 1701 1710 1719 1728
CTA TGG TCT AAC AAG AGG GAC ACT GGA CTT TGG TTT GCT CCT GCT GAG TGG AAA
Leu Trp Ser Asn Lys Arg Asp Thr Gly Leu Trp Phe Ala Pro Ala Glu Trp Lys

1737 1746 1755 1764 1773 1782
GAG TGT AGG CTT GGC ATT CAG CTC TTA CCA TTG GCT CCT ATT TCT GAA GCC ATT
Glu Cys Arg Leu Gly Ile Gln Leu Leu Pro Leu Ala Pro Ile Ser Glu Ala Ile

1791 1800 1809 1818 1827 1836
TTC TCC AAT GTT GAC TTT GTA AAG GAG CTT GTG GAG TGG ACT TTG CCT GCT TTG
Phe Ser Asn Val Asp Phe Val Lys Glu Leu Val Glu Trp Thr Leu Pro Ala Leu

1845 1854 1863 1872 1881 1890
GAT AGG GAG GGT GGT GTT GGT GAA GGA TGG AAG GGG TTT GTG TAT GCC CTT GAA

Asp Arg Glu Gly Gly Val Gly Glu Gly Trp Lys Gly Phe Val Tyr Ala Leu Glu

1899 1908 1917 1926 1935 1944
GGG GTT TAT GAC AAT GAA AGT GCA CTG CAG AAG ATA AGA AAC CTG AAA GGT TTT
Gly Val Tyr Asp Asn Glu Ser Ala Leu Gln Lys Ile Arg Asn Leu Lys Gly Phe

1953 1962 1971 1980 1989 1998
GAT GGT GGA AAC TCT TTG ACC AAT CTC TTG TGG TGG ATT CAT AGC AGA AGT GAT
Asp Gly Gly Asn Ser Leu Thr Asn Leu Leu Trp Trp Ile His Ser Arg Ser Asp

2004
GAA TAG
Glu

INFORMATION FOR SEQ ID NO: 3
LENGTH: 54 base pairs
TYPE: nucleic acid
STRANDEDNESS: single
TOPOLOGY: linear
MOLECULE TYPE: Other nucleic acid, synthetic DNA
SEQUENCE DESCRIPTION: SEQ ID NO: 3:
GGAATTCGAG CTCGGTACCC GGGGGATCCT CTAGAGTCGA CCTGCAGGCA TGCA

INFORMATION FOR SEQ ID NO: 4
LENGTH: 58 base pairs
TYPE: nucleic acid
STRANDEDNESS: single
TOPOLOGY: linear
MOLECULE TYPE: Other nucleic acid, synthetic DNA

SEQUENCE DESCRIPTION: SEQ ID NO: 4:

CCTTAAGCTC GAGCCATGGG CCCCCTAGGA GATCTCAGCT GGACGTCCGT ACGTTCGA

## Claims

1. A glucan elicitor receptor having an amino acid sequence as substantially shown in SEQ ID NO:1.

2. A DNA molecule containing a nucleotide sequence coding for a glucan elicitor receptor having an amino acid sequence as substantially shown in SEQ ID NO:1, or fragments thereof.

3. The DNA molecule of claim 2, wherein the nucleotide sequence coding for a glucan elicitor receptor is as shown in SEQ ID NO:2, or fragments thereof.

4. A DNA molecule containing a nucleotide sequence coding for a glucan elicitor receptor, which is incorporated in plasmid pER23-1, or fragments thereof.

5. A vector containing the DNA molecule coding for a glucan elicitor receptor of any one of claims 2-4 or a fragment thereof.

6. A plant cell transformed with the DNA molecule coding for a glucan elicitor receptor of any one of claims 2-4 or a fragment thereof.
